# EUROPEAN PATENT APPLICATION

(11) **EP 4 721 793 A1**
(43) Date of publication of application: **08.04.2026**
(21) Application number: 24811240.1
(22) Date of filing: 27.03.2024
(51) Int. Cl.: A61M 15/00

(54) **INHALER**

(30) Priority: 24.05.2023 KR 20230066821
(71) Applicant: KT&G Corporation, Daedeok-gu Daejeon 34337 (KR)
(72) Inventor: KIM, Moonwon, Daejeon 34128 (KR); JUNG, Yongmi, Daejeon 34128 (KR); SHIN, Jun Won, Daejeon 34128 (KR)
(74) Representative: Ter Meer Steinmeister & Partner
(86) International application number: PCT/KR2024/003862
(87) International publication number: WO 2024/242301

(57) **Abstract**

An inhaler according to an embodiment comprises: a mouthpiece including a capsule containing a functional material therein; a housing having one end opened to allow the mouthpiece to be inserted therein and having formed therein a space extending in a cylindrical shape to the other end; a piercing portion which is disposed in the housing and extends from the other end of the housing toward one end; and a sliding portion to which the mouthpiece is coupled and which slidably moves inside the housing, wherein a guide for coupling the mouthpiece and a guide for perforating the capsule by using the piercing portion may be separately formed in the housing.

## Description

### TECHNICAL FIELD

An inhaler is disclosed.

### BACKGROUND ART

A conventional dry powder inhaler is intended to transfer a single-dose, metered dose of a pharmacological agent into the body and has been used primarily to treat patients with asthma and chronic obstructive pulmonary disease (COPD). Finely dried powders of 5 micrometers (µm) to 10 µm or less are used for efficient absorption of the agent into the body. In addition, a determined amount of drug may be filled into a hard capsule for quantitative delivery.

The above description is information the inventor(s) acquired during the course of conceiving the present disclosure, or already possessed at the time, and is not necessarily art publicly known before the present application was filed.

Prior art document: Korea Patent Publication No. 10-2006-0126490

### DISCLOSURE OF THE INVENTION

### TECHNICAL GOALS

Embodiments are to provide an inhaler that is easy to carry for timely inhalation of a functional material, may store a capsule including the functional material, and is easy to replace the capsule by attaching or detaching a mouthpiece.

The technical goals obtainable from the embodiments are not limited to the above-mentioned technical goals, and other unmentioned technical goals may be clearly understood from the following description by one of ordinary skill in the art to which the present disclosure pertains.

### TECHNICAL SOLUTIONS

An inhaler according to an embodiment to achieve the goals includes a mouthpiece including a capsule containing a functional material, a housing having one end open so that the mouthpiece is inserted to an inside and a space formed therein that extends to the other end in a cylindrical shape, a piercing portion arranged inside the housing and extending from the other end of the housing toward the one end, and a sliding portion to which the mouthpiece is coupled and configured to slide inside the housing, wherein a guide for coupling the mouthpiece and a guide for perforating the capsule by the piercing portion may be individually formed inside the housing.

The inhaler may further include a storage forming an openable internal space at a position spaced apart from a space where the mouthpiece is coupled in the housing, wherein a plurality of capsules may be accommodated in the storage.

The housing may include a first guide groove extending from the one end of the housing to a first point spaced apart towards the other end, a second guide groove extending from the first point to a second point spaced apart in a circumferential direction of the housing, and a third guide groove extending from the second point to a third point adjacent to the other end of the housing, wherein the mouthpiece coupled to the first guide groove may be restricted from sliding below the first point.

The mouthpiece that has reached the first point of the first guide groove may be rotatable along the second guide groove, and the mouthpiece that has reached the second point may be allowed to slide to the third point.

The inhaler may further include a guide rail extending from a point at a same height as the first point or the second point to the other end of the housing and protruding toward the inside of the housing, wherein the guide rail may be configured to restrict the mouthpiece that has reached the first point from sliding below the first point, and the guide rail may be coupled to the mouthpiece when the mouthpiece moves toward the other end of the housing along the third guide groove.

The mouthpiece may include a first coupling member protruding in a radial direction from the other end of the mouthpiece and a second coupling member that is a groove spaced apart from the first coupling member in a circumferential direction and extending from one end of the mouthpiece to the other end, wherein the first coupling member may be combinable with the first guide groove, the second guide groove, or the third guide groove, and the second coupling member may be combinable with the guide rail.

The mouthpiece may include a receiving member formed adjacent to the other end of the mouthpiece as a cavity for accommodating the capsule inside the mouthpiece and a bottleneck member formed adjacent to the receiving member and having a diameter smaller than the receiving member, wherein the capsule accommodated in the receiving member may be restricted from moving toward the one end of the mouthpiece by the bottleneck member.

The sliding portion may include a moving member having a cylindrical body that slides inside the housing and a plurality of seating members that is spaced apart from each other in a circumferential direction on one surface of the moving member and protrudes toward the one end of the housing, wherein the seating member may be formed as a step with a lower protrusion height as the seating member approaches the center, and the other end of the mouthpiece may be seated on the seating member.

The inhaler may further include a plurality of guide grooves respectively formed in a side surface of the moving member and respectively arranged between two seating members that are spaced apart, wherein the guide grooves may be coupled to a guide rail formed on the inside of the housing.

The inhaler may further include a through hole penetrating the moving member in a same direction as a moving direction of the moving member, wherein, when the moving member slides in a direction from the one end of the housing toward the other end, the piercing portion may pass through the through hole.

The through hole may have a diameter of an opening adjacent to the other end of the housing that is greater than a diameter of an opening adjacent to the one end of the housing.

The inhaler may further include a spring having one end coupled to the sliding portion and the other end coupled to the other end of the housing, wherein the spring contracts when the sliding portion may be pressured and moves toward the other end of the housing, and when the sliding portion is not pressured, the spring may be configured to support the sliding portion to be located at a position spaced apart from the piercing portion toward the one end of the housing.

The inhaler may further include a stopper mounted on one side of the housing and configured to restrict a movement distance of the sliding portion, wherein the stopper may be positioned inside the housing so that one end is positioned outside the housing and the other end is positioned further inside than an outer circumference of the sliding portion.

The stopper may have an air inlet penetrating inside the stopper, and the air inlet may connect the inside and the outside of the housing to air.

### EFFECTS OF THE INVENTION

According to an embodiment, an inhaler is easy to carry for timely inhalation of a functional material, may store a capsule including the functional material, and is easy to replace the capsule by attaching or detaching a mouthpiece.

The effects of the inhaler according to an embodiment are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 is a perspective view of an inhaler according to an embodiment.
FIG. 2 is a cross-sectional view of an inhaler according to an embodiment.
FIG. 3 is a cross-sectional view of an inhaler according to an embodiment in which a capsule is perforated by a piercing portion.
FIG. 4 illustrates a housing of an inhaler according to an embodiment.
FIG. 5 illustrates a mouthpiece of an inhaler according to an embodiment.
FIG. 6 is a perspective view of a sliding portion of an inhaler according to an embodiment.
FIG. 7 is a cross-sectional view of a sliding portion of an inhaler according to an embodiment.

The accompanying drawings illustrate preferred embodiments of the present invention and are provided together with the detailed description for better understanding of the technical idea of the present invention. Therefore, the present invention should not be construed as being limited to the embodiments set forth in the drawings.

### BEST MODE FOR CARRYING OUT THE INVENTION

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe components of the embodiments. These terms are used only for the purpose of discriminating one component from another component, and the nature, the sequences, or the orders of the components are not limited by the terms. When one component is described as being "connected", "coupled", or "attached" to another component, it should be understood that one component may be connected or attached directly to the other component, and an intervening component may also be "connected", "coupled", or "attached" to the components.

The same name may be used to describe an element included in the embodiments described above and an element having a common function. Unless otherwise mentioned, the descriptions of the embodiments may be applicable to the following embodiments and thus, duplicated descriptions will be omitted for conciseness.

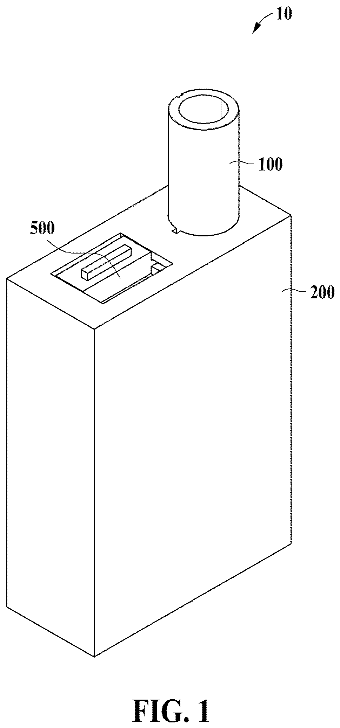

FIG. 1 is a perspective view of an inhaler 10 according to an embodiment.

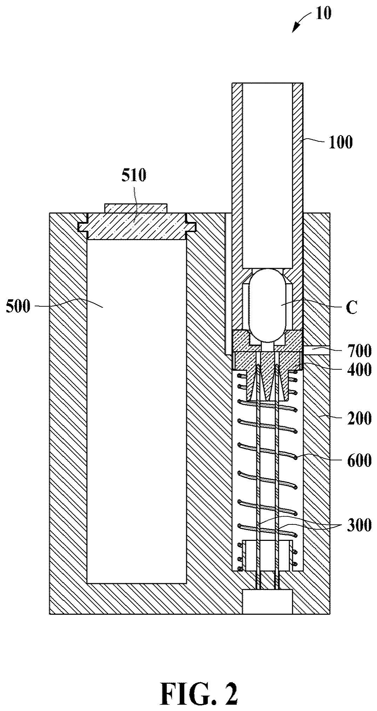

FIG. 2 is a cross-sectional view of the inhaler 10 according to an embodiment.

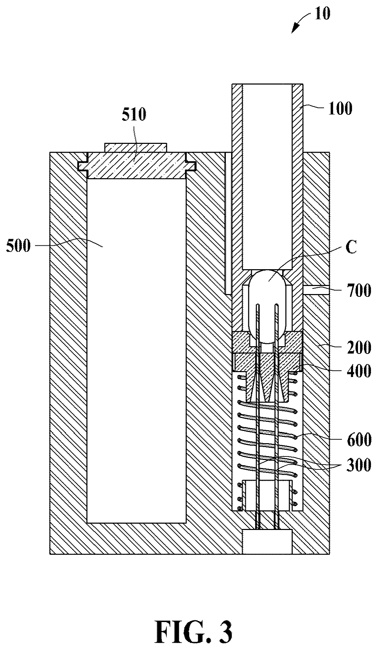

FIG. 3 is a cross-sectional view of the inhaler 10 according to an embodiment in which a capsule C is perforated by a piercing portion 300.

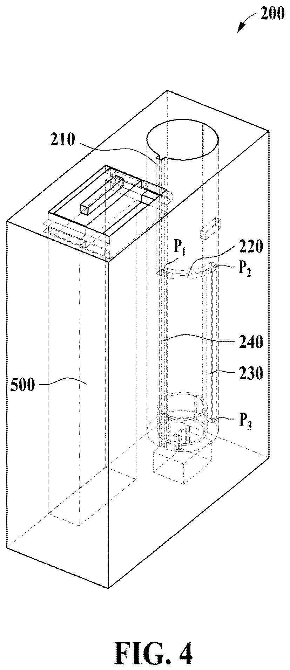

FIG. 4 illustrates a housing 200 of the inhaler 10 according to an embodiment.

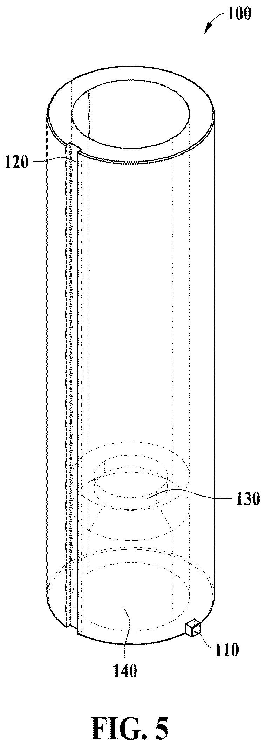

FIG. 5 illustrates a mouthpiece 100 of the inhaler 10 according to an embodiment.

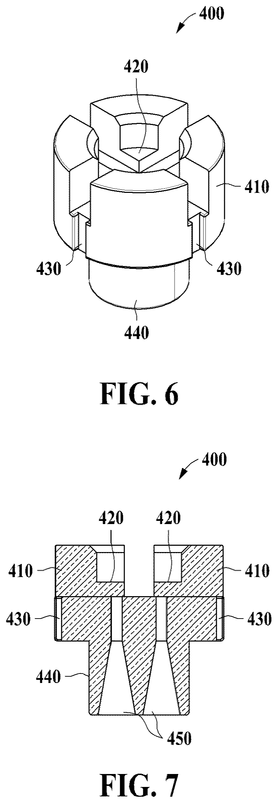

FIG. 6 is a perspective view of a sliding portion 400 of the inhaler 10 according to an embodiment.

FIG. 7 is a cross-sectional view of the sliding portion 400 of the inhaler 10 according to an embodiment.

Referring to FIGS. 1 to 3, the inhaler 10 according to an embodiment may include the mouthpiece 100, the housing 200, the piercing portion 300, the sliding portion 400, and a storage 500. Elements included in the inhaler 10 according to an embodiment may be materials such as polylactic acid (PLA), acrylonitrile butadiene styrene copolymer (ABS), polycarbonate (PC), polypropylene (PP), etc. or may also be other biodegradable (eco-friendly) materials or metals such as aluminum. The inhaler 10 may also be manufactured using a three-dimensional (3D) printer.

The mouthpiece 100 may be formed in a cylindrical shape extending in a longitudinal direction, and an air inlet may be formed in a side surface of the mouthpiece 100. One end of the mouthpiece 100 may be in a shape suitable for a user to put in the mouth and may preferably be in a shape such as a circle or an oval. The mouthpiece 100 may be in a shape suitable for storing the capsule C and may have an internal space larger than the capsule C. In this internal space, a spiral structure may be formed on an inner wall so that a rotational airflow is formed near the capsule C. The capsule C including a functional material may be inserted to the other end of the mouthpiece 100. The size of the capsule C may be, for example, size 0, 1, 2, 3, 4, 5, etc. and preferably size 3. The functional material may include, for example, a material such as nicotine, nicotine salt, caffeine, taurine, and vitamin, a preference inhalation material, and other pharmacological substances. The functional material may be contained in the capsule C in a dry powder form, for example. Here, the size of a dry powder may be about 5 to 10 micrometers (µm) and may include menthol particles of 20 µm or more. This dry powder may or may not have a carrier. Here, the size of the carrier may be about 20 to 50 µm or more and may be about 100 µm. One end of the mouthpiece 100 that the user touches may have a mesh net for separating particles from the capsule C. The mesh net may include PLA or the like, a material that is the same as the material of the housing 200, or a chemically stable metal having no inhalation toxicity, such as stainless steel. After the powder inside the capsule C is exhausted, the capsule C may be replaced by separating the mouthpiece 100 from the housing 200.

The housing 200 may be provided in various shapes, such as a circle or a square, that are easy for the user to hold in the hand. The size of the housing 200 may be, for example, 30 to 50 millimeters (mm) in length, 10 to 30 mm in width, and 50 to 70 mm in height. The housing 200 may have a cylindrical space formed therein that has one end open and extends from the one end to the other end. The mouthpiece 100 may be inserted to the inside of the housing 200 through the one end. A guide for coupling the mouthpiece 100 to the one end of the housing 200 may be formed inside the housing 200. In addition, a guide different from the aforementioned guide may be further formed inside the housing 200. This guide may be for guiding a movement path of the mouthpiece 100 when the capsule C is to be perforated by the piercing portion 300. This guide may be formed separately from the guide, which is extended from the one end, to allow the mouthpiece 100 to move further toward the other end of the housing 200 to reach the piercing portion 300.

Referring to FIGS. 2 and 3, the piercing portion 300 may be arranged inside the housing 200. The piercing portion 300 may be arranged at the other end of the housing 200. The piercing portion 300 may extend toward the one end. The piercing portion 300 may perforate the capsule C. The piercing portion 300 may also be coupled to the other end of the housing 200 in a replaceable structure for separate maintenance. For example, the piercing portion 300 may be provided as a needle. The piercing portion 300 may be provided with one needle, but preferably, two needles may be arranged in parallel. The two needles may have the same or different lengths, and needles of different lengths may have various diameters. A diameter of the needle may be provided as, for example, 0.5 to 1.2 mm and preferably 0.7 mm.

The sliding portion 400 may be arranged to be slidable inside the housing 200. As described above, the housing 200 may have a rough structure formed inside to prevent the sliding portion 400 from deviating from the path. Through this structure, when the mouthpiece 100 and the sliding portion 400 are lowered to perforate the capsule C, the sliding portion 400 may move parallel to the piercing portion 300. An end portion of the mouthpiece 100 may be coupled to the sliding portion 400. When the sliding portion 400 slides, the mouthpiece 100 may also move together.

The storage 500 may form an internal space in the housing 200 at a position separated from the space where the mouthpiece is coupled. A plurality of capsules C may be accommodated in the storage 500. For example, 1 to 20 capsules C may be stored in the storage 500.

The storage 500 may also be formed in an openable structure. A door 510 may be formed at one end of the storage 500. The door 510 may be implemented in a slide structure and may be provided in a form of opening and closing a lid or a button-type opening and closing structure.

Referring to FIGS. 2 and 3, the inhaler 10 according to an embodiment may further include a spring 600.

The spring 600 may have one end coupled to the sliding portion 400 and the other end coupled to the other end of the housing 200. For example, the piercing portion 300 including two needles may be arranged at a distance that is less than a diameter of the spring 600 and may be arranged to be surrounded by the spring 600.

Referring to FIG. 2, when the mouthpiece 100 or the sliding portion 400 is not pressured by an external force, the spring 600 may support the sliding portion 400 to be located at a position spaced from the piercing portion 300 toward the one end of the housing 200.

Referring to FIG. 3, when the mouthpiece 100 or the sliding portion 400 is pressured and moves toward the other end of the housing 200, the spring 600 may be contracted. Accordingly, the piercing portion 300 may penetrate the sliding portion 400 and the mouthpiece 100 so that the capsule C accommodated in the mouthpiece 100 may be perforated.

The capsule C may contain a functional material in a dry powder form, for example. As described above, since the capsule C is perforated by the piercing portion 300, the functional material in the capsule C may be inhalable by the user. In addition, since the user inhales the functional material while the capsule C is fastened to the mouthpiece 100, suction resistance may increase.

Referring to FIG. 4, the housing 200 may have a first guide groove 210, a second guide groove 220, and a third guide groove 230 formed in a longitudinal direction in the cylindrical space having one end open and extends from the one end to the other end.

The first guide groove 210 may extend from the one end of the housing 200 to a first point P1 spaced apart towards the other end.

The second guide groove 220 may extend from the first point P1 to a second point P2 spaced apart in a circumferential direction of the housing 200.

The third guide groove 230 may extend from the second point P2 to a third point P3 adjacent to the other end of the housing 200.

Referring again to FIG. 4, the housing 200 may further have a guide rail 240 formed therein.

The guide rail 240 may extend from any point at the same height as the first point P1 or the second point P2 to the other end of the housing 200. The guide rail 240 may protrude toward the inside of the housing 200.

The guide rail 240 may restrict the mouthpiece 100 that has reached the first point P1 from sliding below the first point P1. In addition, the guide rail 240 may be coupled to the mouthpiece 100 when the mouthpiece 100 moves toward the other end of the housing 200 along the third guide groove 230.

Referring to FIG. 5, the mouthpiece 100 may include a first coupling member 110 formed on the outside and a second coupling member 120.

The first coupling member 110 may protrude in a radial direction from the other end of the mouthpiece 100. The first coupling member 110 may be coupled to the first guide groove 210, the second guide groove 220, or the third guide groove 230.

The second coupling member 120 may be formed as a groove that is spaced from the first coupling member 110 in the circumferential direction and extends from the one end of the mouthpiece 100 to the other end. The second coupling member 120 may be coupled to the guide rail 240.

The first coupling member 110 of the mouthpiece 100 may be coupled from the one end of the housing 200 to the first guide groove 210 and may move toward the first point P1. Accordingly, the mouthpiece 100 may be inserted into the housing 200 to the first point P1. Here, the mouthpiece 100 may be prevented from rotating by the coupling of the first guide groove 210 with the first coupling member 110.

In a state in which the first coupling member 110 is coupled to the first guide groove 210, as described above, the sliding movement of the mouthpiece 100 passing the first point P1 toward the other end of the housing 200 may be restricted.

In order for the capsule C in the mouthpiece 100 to be perforated by the piercing portion 300, the mouthpiece 100 may need to move further toward the other end of the housing 200. To this end, the first coupling member 110 may move from the first point P1 to the second point P2 that is located at the same height as the first point and spaced apart from the first point P1 in the circumferential direction.

When the first coupling member 110 reaches the second point, the first coupling member 110 may be movable along the third guide groove 230, and the second coupling member 120 may be located at a position corresponding to the guide rail 240 so that the second coupling member 120 may be coupled with the guide rail 240. When pressure is applied to the mouthpiece 100 arranged in such a state, the mouthpiece 100 may slide toward the other end of the housing 200 passing the first point P1 and the second point P2. Accordingly, the capsule C may reach the piercing portion 300 and be perforated.

As described above, the housing 200 may be provided with the first guide groove 210, the second guide groove 220, the third guide groove 230, and the guide rail 240 that are provided separately, thereby facilitating the coupling of the mouthpiece 100 to the housing 200 and preventing the mouthpiece 100 from rotating or being separated from the housing 200 after the coupling. In addition, the rotation of the mouthpiece 100 may be allowed only at a specific point in which the second guide groove 220 is located, and the mouthpiece 100 may be guided to move parallel to the piercing portion 300, thereby facilitating the perforation of the capsule C.

Referring again to FIG. 5, the mouthpiece 100 may further include a receiving member 140 and a bottleneck member 130.

The receiving member 140 may be a cavity formed adjacent to the other end of the mouthpiece 100. The receiving member 140 may accommodate the capsule C.

The bottleneck member 130 may be formed adjacent to the receiving member 140. The bottleneck member 130 may be formed to have a diameter smaller than the receiving member 140. Due to the shape of the bottleneck member 130, the capsule C accommodated in the receiving member 140 may be restricted from moving toward the one end of the mouthpiece 100. In addition, when the piercing portion 300 perforates the capsule C, the capsule C may be prevented from being twisted due to the bottleneck member 130.

Referring to FIG. 6, the sliding portion 400 may include a moving member 410, a seating member 420, a guide groove 430, a protruding member 440, and a through hole 450.

The moving member 410 may have a cylindrical body. The moving member 410 may slide inside the housing 200.

The seating member 420 may be formed on one surface of the moving member 410. A plurality of seating members 420 may be provided. Here, the plurality of seating members 420 may be spaced apart from each other in a circumferential direction of the moving member. The seating member 420 may protrude from one surface of the moving member 410 toward the one end of the housing 200. Alternatively, the seating member 420 may be formed so that a center portion is recessed in a direction opposite to a direction toward the one end of the housing 200 from the one surface of the moving member 410.

The seating member 420 may be formed, for example, as a two-step. As shown in FIGS. 6 and 7, the seating member 420 may be formed as a step in which a protrusion height from the one surface of the moving member 410 becomes low as the seating member 420 approaches the center. The capsule C may be seated on the seating member 420. Due to the step shape of the seating member 420, the capsule C may not contact the one surface of the moving member 410 so that an air inlet space may be secured between the one surface and the capsule C. Accordingly, the airflow may be smooth, so the inhaler 10 according to an embodiment may allow the user to easily inhale the functional material inside the capsule C while the capsule C is fastened to the seating member 420.

In the two-step structure of the seating member 420, at least a portion (e.g., an upper portion of the seating member 420 shown in FIG. 7) of the two-step structure on a side facing the one end of the housing 200 may have an inclined structure. This inclined structure may smoothly induce vertical standing of the capsule C. Through the inclined structure, a diameter of an accommodation space formed by the seating member 420 may be configured to be greater than a diameter of the capsule C, and sufficient space may be secured to accommodate the capsule C, thereby smoothly inducing rotation of the capsule C when air is introduced through inhalation.

Furthermore, an additional step may be formed in an upper end portion (e.g., the upper portion of the seating member 420 shown in FIG. 7) of the seating member 420. The mouthpiece 100 may be seated on the additional step, and the mouthpiece 100 may not contact the one surface of the moving member 410.

The guide groove 430 may be formed in a side surface of the moving member 410. A plurality of guide grooves 430 may be provided, and each guide groove 430 may be arranged between two seating members 420 spaced apart. At least one of the plurality of guide grooves 430 may be coupled to the guide rail 240. Accordingly, the sliding portion 400 may be moved along the guide rail 240 when moving inside the housing 200 and may be prevented from rotating during the movement.

The protruding member 440 may be formed on the other surface of the moving member 410. The protruding member 440 may protrude in a cylindrical form from the other surface of the moving member 410 toward the other end of the housing 200. A diameter of the protruding member 440 may be formed smaller than the diameter of the moving member 410. One end of the spring 600 may be coupled to the protruding member 440. Accordingly, the moving member 410 may efficiently compress the spring 600.

Referring to FIG. 7, the through hole 450 may be formed to penetrate the moving member 410 in the same direction as the moving direction of the moving member 410. The piercing portion 300 may pass through the through hole 450 when the moving member 410 slides from the one end of the housing 200 toward the other end. The through hole 450 may be formed in a trumpet shape. For example, the through hole 450 may have a diameter of an opening adjacent to the other end of the housing 200 that is greater than a diameter of an opening adjacent to the one end of the housing 200. This shape of the through hole 450 may allow the piercing portion 300 to smoothly pass through the through hole 450 so that the piercing portion 300 may easily reach the capsule C.

The inhaler 10 according to an embodiment may further include a stopper (not shown). Referring to FIGS. 2 and 3, an air hole 700 of a small size may be formed on the side surface of the housing 200 to form smooth airflow when forming a negative pressure. The stopper may be inserted to the air hole 700 of the housing 200. The stopper may be inserted so that one end is positioned on the outside of the housing 200 and the other end is positioned on the inside of the housing 200 of the sliding portion 400. Here, the other end of the stopper may be inserted to the housing 200 so that the other end is positioned further inside than an outer circumference of the moving member 410. This stopper may prevent shock due to elasticity of the spring 600 when the sliding portion 400 moves up and down.

In addition, the stopper may have an air inlet formed through the inside of the stopper. The air inlet may connect an air inlet space between the aforementioned mouthpiece 100 and the sliding portion 400 to the outside of the housing 200. That is, the air inlet of the stopper may serve as an air hole of the housing 200. Accordingly, the inhaler 10 according to an embodiment may form smooth airflow from the outside to the air inlet space and may allow the user to easily inhale the functional material inside the capsule C.

As described above, the inhaler 10 according to an embodiment may be easy to carry for timely inhalation of the functional material, may store the plurality of capsules C containing the functional material, and may allow easy replacement of the capsule C by attaching and detaching the mouthpiece 100.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the scope of the claims and their equivalents. For example, suitable results may be achieved if the described techniques are performed in a different order and/or if components in a described system, architecture, device, or circuit are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a mouthpiece comprising a capsule containing a functional material;
a housing having one end open so that the mouthpiece is inserted to an inside and a space formed therein that extends to the other end in a cylindrical shape;
a piercing portion arranged inside the housing and extending from the other end of the housing toward the one end; and
a sliding portion to which the mouthpiece is coupled and configured to slide inside the housing,
wherein a guide for coupling the mouthpiece and a guide for perforating the capsule by the piercing portion are individually formed inside the housing.

2. The inhaler of claim 1, further comprising a storage forming an openable internal space at a position spaced apart from a space where the mouthpiece is coupled in the housing, wherein a plurality of capsules is accommodated in the storage.

3. The inhaler of claim 1, wherein the housing comprises:
a first guide groove extending from the one end of the housing to a first point spaced apart towards the other end;
a second guide groove extending from the first point to a second point spaced apart in a circumferential direction of the housing; and
a third guide groove extending from the second point to a third point adjacent to the other end of the housing,
wherein the mouthpiece coupled to the first guide groove is restricted from sliding below the first point.

4. The inhaler of claim 3, wherein the mouthpiece that has reached the first point of the first guide groove is rotatable along the second guide groove, and the mouthpiece that has reached the second point is allowed to slide to the third point.

5. The inhaler of claim 3, further comprising:
a guide rail extending from a point at a same height as the first point or the second point to the other end of the housing and protruding toward the inside of the housing,
wherein the guide rail is configured to restrict the mouthpiece that has reached the first point from sliding below the first point, and
the guide rail is coupled to the mouthpiece when the mouthpiece moves toward the other end of the housing along the third guide groove.

6. The inhaler of claim 5, wherein the mouthpiece comprises:
a first coupling member protruding in a radial direction from the other end of the mouthpiece; and
a second coupling member that is a groove spaced apart from the first coupling member in a circumferential direction and extending from one end of the mouthpiece to the other end,
wherein the first coupling member is combinable with the first guide groove, the second guide groove, or the third guide groove, and the second coupling member is combinable with the guide rail.

7. The inhaler of claim 6, wherein the mouthpiece comprises:
a receiving member formed adjacent to the other end of the mouthpiece as a cavity for accommodating the capsule inside the mouthpiece; and
a bottleneck member formed adjacent to the receiving member and having a diameter smaller than the receiving member, wherein the capsule accommodated in the receiving member is restricted from moving toward the one end of the mouthpiece by the bottleneck member.

8. The inhaler of claim 1, wherein the sliding portion comprises:
a moving member having a cylindrical body that slides inside the housing; and
a plurality of seating members that is spaced apart from each other in a circumferential direction on one surface of the moving member and protrudes toward the one end of the housing, wherein the seating member is formed as a step with a lower protrusion height as the seating member approaches the center, and the other end of the mouthpiece is seated on the seating member.

9. The inhaler of claim 8, further comprising a plurality of guide grooves respectively formed in a side surface of the moving member and respectively arranged between two seating members that are spaced apart, wherein the guide grooves are coupled to a guide rail formed on the inside of the housing.

10. The inhaler of claim 8, further comprising a through hole penetrating the moving member in a same direction as a moving direction of the moving member, wherein, when the moving member slides in a direction from the one end of the housing toward the other end, the piercing portion passes through the through hole.

11. The inhaler of claim 10, wherein the through hole has a diameter of an opening adjacent to the other end of the housing that is greater than a diameter of an opening adjacent to the one end of the housing.

12. The inhaler of claim 1, further comprising a spring having one end coupled to the sliding portion and the other end coupled to the other end of the housing, wherein the spring contracts when the sliding portion is pressured and moves toward the other end of the housing, and when the sliding portion is not pressured, the spring is configured to support the sliding portion to be located at a position spaced apart from the piercing portion toward the one end of the housing.

13. The inhaler of claim 1, further comprising a stopper mounted on one side of the housing and configured to restrict a movement distance of the sliding portion, wherein the stopper is positioned inside the housing so that one end is positioned outside the housing and the other end is positioned further inside than an outer circumference of the sliding portion.

14. The inhaler of claim 13, wherein the stopper has an air inlet penetrating inside the stopper, and the air inlet connects the inside and the outside of the housing to air.
